(19) Europäisches Patentamt / European Patent Office / Office européen des brevets

(11) **EP 2 739 211 B1**

(12) **FASCICULE DE BREVET EUROPEEN**

(45) Date de publication et mention de la délivrance du brevet:
**05.09.2018 Bulletin 2018/36**

(21) Numéro de dépôt: **12743132.8**

(22) Date de dépôt: **26.07.2012**

(51) Int Cl.:
***A61B 8/08*** (2006.01)

(86) Numéro de dépôt international:
**PCT/EP2012/064738**

(87) Numéro de publication internationale:
**WO 2013/017532 (07.02.2013 Gazette 2013/06)**

(54) **PROCEDE POUR DETERMINER EN TEMPS-REEL UNE PROBABILITE DE PRESENCE D'UN TISSU BIOLOGIQUE CIBLE EN REGARD D'UN TRANSDUCTEUR ULTRASONORE**

VERFAHREN ZUR ECHTZEITBESTIMMUNG DES VORHANDENSEINS VON BIOLOGISCHEM GEWEBE GEGENÜBER EINEM ULTRASCHALLWANDLER

METHOD FOR DETERMINING, IN REAL TIME, THE PROBABILITY THAT TARGET BIOLOGICAL TISSUE IS OPPOSITE AN ULTRASONIC TRANSDUCER

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **03.08.2011 FR 1157119**

(43) Date de publication de la demande:
**11.06.2014 Bulletin 2014/24**

(73) Titulaire: **Echosens**
**75013 Paris (FR)**

(72) Inventeurs:
• **SANDRIN, Laurent**
**92240 L'Hay Les Roses (FR)**
• **MIETTE, Véronique**
**94800 Villejuif (FR)**
• **OUDRY, Jennifer**
**92240 Malakoff (FR)**
• **AUDIERE, Stéphane**
**94800 Villejuif (FR)**
• **MOFID, Yassine**
**37550 Saint Avertin (FR)**

(74) Mandataire: **Lebkiri, Alexandre**
**Cabinet Camus Lebkiri**
**25, Rue de Maubeuge**
**75009 Paris (FR)**

(56) Documents cités:
• **SCHMITZ G ET AL: "Tissue characterization of the prostate using Kohonen-maps",** ULTRASONICS SYMPOSIUM, 1994. PROCEEDINGS., 1994 IEEE CANNES, FRANCE 1-4 NOV. 1994, NEW YORK, NY, USA,IEEE, US, 31 octobre 1994 (1994-10-31), pages 1487-1490VOL.3, XP032084618, DOI: 10.1109/ULTSYM.1994.401872 ISBN: 978-0-7803-2012-3
• **SCHEIPERS U ET AL: "Ultrasonic multifeature tissue characterization for prostate diagnostics",** ULTRASOUND IN MEDICINE AND BIOLOGY, NEW YORK, NY, US, vol. 29, no. 8, 1 août 2003 (2003-08-01), pages 1137-1149, XP004451153, ISSN: 0301-5629, DOI: 10.1016/S0301-5629(03)00062-0
• **YASSER M KADAH ET AL: "Classification Algorithms for Quantitative Tissue Characterization of Diffuse Liver Disease from Ultrasound Images",** IEEE TRANSACTIONS ON MEDICAL IMAGING, IEEE SERVICE CENTER, PISCATAWAY, NJ, US, vol. 15, no. 4, 1 août 1996 (1996-08-01), XP011035565, ISSN: 0278-0062
• **CHOU Y-H ET AL: "Stepwise logistic regression analysis of tumor contour features for breast ultrasound diagnosis",** ULTRASOUND IN MEDICINE AND BIOLOGY, NEW YORK, NY, US, vol. 27, no. 11, 1 novembre 2001 (2001-11-01), pages 1493-1498, XP004327047, ISSN: 0301-5629, DOI: 10.1016/S0301-5629(01)00466-5

**Description**

L'invention concerne un procédé pour déterminer en temps-réel une probabilité de présence d'un tissu biologique cible en regard d'un transducteur ultrasonore. Le transducteur ultrasonore peut, par exemple, appartenir à une sonde de mesure dite « aveugle » ou « partiellement aveugle ». On entend par « aveugle » que le dispositif de mesure auquel est reliée la sonde de mesure est dépourvu d'un mode d'imagerie temps réel. En outre, on entend par « partiellement aveugle » que le mode d'imagerie du dispositif de mesure est limité à une ligne ultrasonore monodimensionnelle (1D) de type A-SCAN associée éventuellement à un mode de type M. La publication SCHMITZ G ET AL, "Tissue Characterization of the Prostate Using Kohonen-Maps", 1994 Ultrasonics Symposium, 1487-1490 (XP 32084618) divulgue un procédé pour déterminer une probabilité de présence d'un tissu biologique cible en regard d'un transducteur ultrasonore, ledit procédé comportant les étapes suivantes: émission, via un transducteur ultrasonore, d'au moins un signal ultrasonore dans un tissu biologique; réception, par ledit transducteur ultrasonore, dudit au moins un signal ultrasonore émis rétrodiffusé par ledit tissu biologique; calcul d'au moins deux paramètres instantanés dudit au moins un signal ultrasonore rétrodiffusé; calcul d'une valeur prédictive de présence d'une signature acoustique d'un tissu biologique cible, ladite valeur prédictive étant calculée via une loi statistique, utilisant lesdits au moins deux paramètres instantanés calculés; estimation d'une probabilité de présence dudit tissu biologique cible en regard dudit transducteur ultrasonore, ladite estimation étant fonction de ladite valeur prédictive calculée. Classiquement, pour mesurer des paramètres quantitatifs ou qualitatifs d'un tissu biologique, une sonde de mesure est positionnée en regard de celui-ci.

Lorsque l'on souhaite positionner une sonde de mesure aveugle ou partiellement aveugle en regard d'un tissu biologique cible, le positionnement peut être réalisé de deux manières:

- un positionnement sans recourir à une sonde d'imagerie annexe, un tel positionnement impose pour l'opérateur de détenir des connaissances en anatomie humaine ou animale,
- un positionnement à l'aide d'une sonde d'imagerie, par exemple d'une sonde d'échographie, différente de la sonde de mesure.

De telles mises en oeuvre entraînent cependant des inconvénients.

En effet, l'utilisation d'une sonde de mesure aveugle sans recourir à une sonde d'imagerie ne permet pas d'assurer avec certitude que la sonde de mesure soit positionnée en regard du tissu biologique cible que l'on souhaite mesurer. Le positionnement de la sonde de mesure est réalisé de manière approximative puisque les connaissances anatomiques sont moyennées sur la population générale. Ainsi, l'anatomie de l'individu sur lequel on souhaite mesurer des paramètres doit être conforme au standard. En effet, si l'anatomie de l'individu est différente de la moyenne, la sonde de mesure ne sera pas positionnée en regard du tissu biologique cible et la mesure obtenue ne sera pas représentative du tissu biologique cible.

Par ailleurs, l'utilisation d'une sonde d'imagerie de façon à faciliter le positionnement de la sonde de mesure en regard du tissu biologique cible, impose de, premièrement, repérer le tissu biologique cible au moyen de la sonde d'imagerie puis, deuxièmement, une fois que le tissu biologique cible est repéré, retirer cette sonde d'imagerie pour la remplacer par une sonde de mesure. Néanmoins, ce remplacement de la sonde d'imagerie par la sonde de mesure ne permet pas d'assurer avec certitude que la sonde de mesure soit positionnée en regard du tissu biologique cible puisque les tissus biologiques d'un individu sont soumis à des mouvements par exemple de type respiratoire.

Une possibilité supplémentaire pour réaliser une mesure quantitative ou qualitative d'un tissu biologique est d'utiliser une sonde apte à imager et apte à mesurer les tissus biologiques. Pour interpréter l'image obtenue à l'aide de cette sonde, il est nécessaire que l'opérateur possède des connaissances accrues, d'une part, dans le domaine de la médecine humaine ou animale et d'autre part, dans le domaine de l'imagerie médicale.

En outre, un opérateur possédant de telles connaissances peut toutefois réaliser des erreurs d'interprétation de l'image utilisée pour le positionnement de la sonde.

Le procédé de l'invention a donc plus particulièrement pour but de remédier aux inconvénients de l'état de la technique. Dans ce contexte, l'invention vise à proposer un procédé pour déterminer une probabilité de présence d'un tissu biologique cible en regard d'un transducteur ultrasonore ne nécessitant pas pour l'opérateur de connaissances accrues dans le domaine de la médecine humaine ou animale ni même dans le domaine de l'instrumentation médicale et évitant en outre une erreur de l'opérateur.

A cette fin, l'invention porte sur un procédé pour déterminer en temps-réel une probabilité de présence d'un tissu biologique cible en regard d'un transducteur ultrasonore, ledit procédé comportant les étapes suivantes :

- émission, via un transducteur ultrasonore, d'au moins un signal ultrasonore dans un tissu biologique ;
- réception, par ledit transducteur ultrasonore, dudit au moins un signal ultrasonore émis rétrodiffusé par ledit tissu biologique ;
- calcul d'au moins deux paramètres instantanés dudit au moins un signal ultrasonore rétrodiffusé ;

- calcul d'une valeur prédictive de présence d'une signature acoustique d'un tissu biologique cible, ladite valeur prédictive étant calculée via une loi statistique utilisant lesdits au moins deux paramètres instantanés calculés ;
- estimation d'une probabilité de présence dudit tissu biologique cible en regard dudit transducteur ultrasonore, ladite estimation étant fonction de ladite valeur prédictive calculée et d'au moins une condition de robustesse basée sur au moins un desdits deux paramètres instantanés calculés.

Il convient de noter que le transducteur ultrasonore utilisé par le procédé peut être de type mono-élément ou multi-éléments.

Un transducteur ultrasonore mono-élément peut émettre et recevoir un unique signal ultrasonore tandis qu'un transducteur ultrasonore multi-éléments peut émettre et recevoir une pluralité de signaux ultrasonores simultanément.

Grâce au procédé de l'invention, on estime en temps réel une probabilité de présence d'un transducteur ultrasonore en regard d'un tissu biologique cible. Cette estimation est réalisée de façon automatique et ne requiert aucune compétence ni même aucune interprétation de l'opérateur mettant en oeuvre le transducteur ultrasonore. L'estimation peut être restituée à l'opérateur sous la forme d'une indication dite sensorielle (visuelle, sonore, tactile (vibration), etc) ou bien directement utilisée pour déclencher une mesure de manière automatique.

Le procédé selon l'invention peut également présenter une ou plusieurs des caractéristiques ci-dessous, considérées individuellement ou selon toutes les combinaisons techniquement possibles.

Dans une mise en oeuvre non limitative du procédé selon l'invention, le calcul d'une valeur prédictive est réalisé via lesdits au moins deux paramètres instantanés calculés et des paramètres instantanés calculés antérieurement mémorisés.

Dans une mise en oeuvre non limitative du procédé selon l'invention, l'estimation fonction de la valeur prédictive calculée est réalisée en comparant la valeur prédictive calculée à une unique ou plusieurs valeurs seuil de prédiction déterminée de sorte que :

lorsque la valeur prédictive calculée est comparée à une unique valeur seuil de prédiction maximum déterminée,

- si la valeur prédictive calculée est strictement inférieure à la valeur seuil de prédiction maximum déterminée alors le transducteur ultrasonore est en regard du tissu biologique cible,
- si la valeur prédictive calculée est supérieure ou égale à la valeur seuil de prédiction maximum déterminée, alors le transducteur ultrasonore est en regard d'un tissu biologique différent du tissu biologique cible ;

lorsque la valeur prédictive calculée est comparée à une valeur seuil de prédiction maximum déterminée et une valeur seuil de prédiction minimum déterminée,

- si la valeur prédictive calculée est inférieure ou égale à la valeur seuil de prédiction minimum déterminée alors le transducteur ultrasonore est en regard du tissu biologique cible,
- si la valeur prédictive calculée est supérieure ou égale à la valeur seuil de prédiction maximum déterminée, alors le transducteur ultrasonore est en regard d'un tissu biologique différent du tissu biologique cible,
- si la valeur prédictive calculée est strictement supérieure à la valeur seuil de prédiction minimum déterminée et strictement inférieure à la valeur seuil de prédiction maximum déterminée alors le transducteur ultrasonore est supposé être en regard du tissu biologique cible. Cette incertitude peut correspondre à une signature acoustique du tissu biologique cible modifiée par une présence d'hétérogénéités engendrée par des métastases ou encore par la présence de vaisseaux sanguins, etc.

Dans une mise en oeuvre non limitative du procédé selon l'invention, les au moins deux paramètres instantanés calculés sont choisis parmi les paramètres suivants :

○ l'énergie du au moins un signal ultrasonore rétrodiffusé,
○ l'atténuation du au moins un signal ultrasonore rétrodiffusé,
○ la linéarité de l'enveloppe du au moins un signal ultrasonore rétrodiffusé,
○ le coefficient d'intercorrélation des signaux ultrasonores rétrodiffusés reçus à deux moments successifs,
○ l'amplitude ou les variations maximum d'amplitude de l'enveloppe du signal ultrasonore rétrodiffusé,
○ la corrélation du au moins un signal ultrasonore rétrodiffusé,
○ la variance spectrale du au moins un signal ultrasonore rétrodiffusé,
○ le coefficient de rétrodiffusion du au moins un signal ultrasonore rétrodiffusé.

Dans une mise en oeuvre non limitative du procédé selon l'invention, la condition de robustesse est :

- si la linéarité de l'enveloppe du au moins un signal ultrasonore rétrodiffusé calculée est supérieure ou égale à une valeur seuil de linéarité déterminée, alors le transducteur ultrasonore est en regard du tissu biologique cible, et/ou
- si l'énergie du au moins un signal ultrasonore rétrodiffusé est supérieure ou égale à une valeur seuil d'énergie déterminée, alors le transducteur ultrasonore est en regard du tissu biologique cible.

Dans une mise en oeuvre non limitative du procédé selon l'invention, quelque soit la valeur prédictive calculée, ladite condition de robustesse est :

- si la linéarité de l'enveloppe du au moins un signal ultrasonore rétrodiffusé calculée est strictement inférieure à la valeur seuil de linéarité déterminée, alors le transducteur ultrasonore est en regard d'un tissu biologique différent du tissu biologique cible,
- si l'énergie du au moins un signal ultrasonore rétrodiffusé est strictement inférieure à la valeur seuil d'énergie déterminée, alors le transducteur ultrasonore est en regard d'un tissu biologique différent du tissu biologique cible.

Dans une mise en oeuvre non limitative du procédé selon l'invention, la loi statistique est une régression logistique de sorte que la valeur prédictive soit calculée via la régression logistique suivante :

$$P = \beta_0 + \beta_1 \cdot Paramètre\_calculé\_1 + ..... + \beta_n \cdot Paramètre\_calculé\_n$$

Dans une mise en oeuvre non limitative du procédé selon l'invention, le procédé comporte une étape supplémentaire d'indication en temps réel d'une probabilité de présence du tissu biologique cible en regard du transducteur ultrasonore. L'indication peut être une indication visuelle, et/ou sonore et/ou tactile. Dans une mise en oeuvre non limitative du procédé selon l'invention, l'indication visuelle est réalisée par un affichage d'un gradient de couleur. Préférentiellement, le gradient de couleur suit une courbe en S.

Dans une mise en oeuvre non limitative du procédé selon l'invention, lorsque le transducteur ultrasonore est en regard du tissu biologique cible, une mesure quantitative et/ou qualitative du tissu biologique cible est déclenchée de façon automatique ou manuelle. Préférentiellement, la mesure est réalisée sur une profondeur déterminée dépendant de la fréquence du au moins un signal ultrasonore émis. Par exemple, cette profondeur déterminée peut être comprise entre 10 et 90 mm ou entre 35 et 75 mm pour un signal ultrasonore de 2,5 Mhz, la profondeur déterminée étant mesurée à partir de la surface de contact entre le transducteur ultrasonore et le tissu biologique en contact.

Dans une mise en oeuvre non limitative du procédé selon l'invention, le procédé comporte une étape supplémentaire de validation de la mesure quantitative et/ou qualitative. Cette validation peut par exemple être effectuée au moyen de la valeur prédictive de présence du tissu biologique cible.

L'invention à également pour objet un dispositif pour déterminer en temps-réel une probabilité de présence d'un tissu biologique cible en regard d'un transducteur ultrasonore, le dispositif comportant au moins un transducteur ultrasonore apte à émettre et recevoir des signaux ultrasonores, le dispositif étant apte à :

- émettre, via le transducteur ultrasonore, au moins un signal ultrasonore dans un tissu biologique ;
- recevoir, par le transducteur ultrasonore, le au moins un signal ultrasonore émis rétrodiffusé par le tissu biologique ;
- calculer au moins deux paramètres instantanés du au moins un signal ultrasonore rétrodiffusé ;
- calculer une valeur prédictive de présence d'une signature acoustique d'un tissu biologique cible, la valeur prédictive étant calculée via une loi statistique utilisant les au moins deux paramètres instantanés calculés ;
- estimer une probabilité de présence du tissu biologique cible en regard du transducteur ultrasonore, l'estimation étant fonction de la valeur prédictive et d'au moins une condition de robustesse basée sur au moins un des deux paramètres calculés,
- indiquer, via un indicateur, en temps réel une probabilité de présence du tissu biologique cible en regard du transducteur ultrasonore.

D'autres caractéristiques et avantages de l'invention ressortiront clairement de la description qui en est donnée ci-après, à titre indicatif et nullement limitatif, en référence aux figures annexées ci-jointes, parmi lesquelles :

- la figure 1 illustre de façon schématique un dispositif pour déterminer en temps-réel une probabilité de présence d'un tissu biologique cible en regard d'un transducteur ultrasonore selon l'invention.
- la figure 2 représente un synoptique des étapes d'un procédé pour déterminer en temps-réel une probabilité de présence d'un tissu biologique cible en regard d'un transducteur ultrasonore conforme à l'invention,
- la figure 3 illustre une étape d'indication visuelle mis en oeuvre par un procédé conforme à l'invention.

Pour des raisons de clarté, seuls les éléments essentiels pour la compréhension de l'invention ont été représentés, et ceci sans respect de l'échelle et de manière schématique.

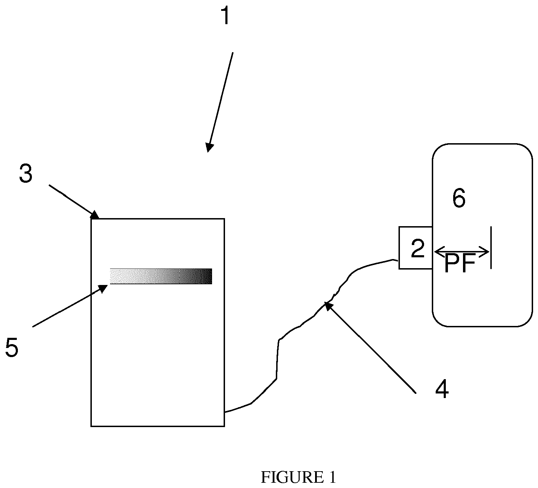

Un dispositif 1 pour déterminer en temps-réel une probabilité de présence d'un tissu biologique cible en regard d'un transducteur ultrasonore conforme à l'invention est représenté sur la figure 1. Le dispositif 1 comporte notamment un transducteur ultrasonore 2 relié à une unité de traitement des informations 3 par une liaison filaire 4. Le dispositif 1 comporte en outre un indicateur 5 en temps réel d'une probabilité de présence d'un tissu biologique cible 6 en regard du transducteur ultrasonore 2. Le fonctionnement de ce dispositif 1 sera détaillé ultérieurement.

Dans l'exemple non limitatif décrit le transducteur ultrasonore 2 utilisé est un transducteur mono-élément. Ainsi, dans une telle réalisation, le transducteur ultrasonore 2 mono-élément peut uniquement émettre et recevoir un signal ultrasonore à la fois.

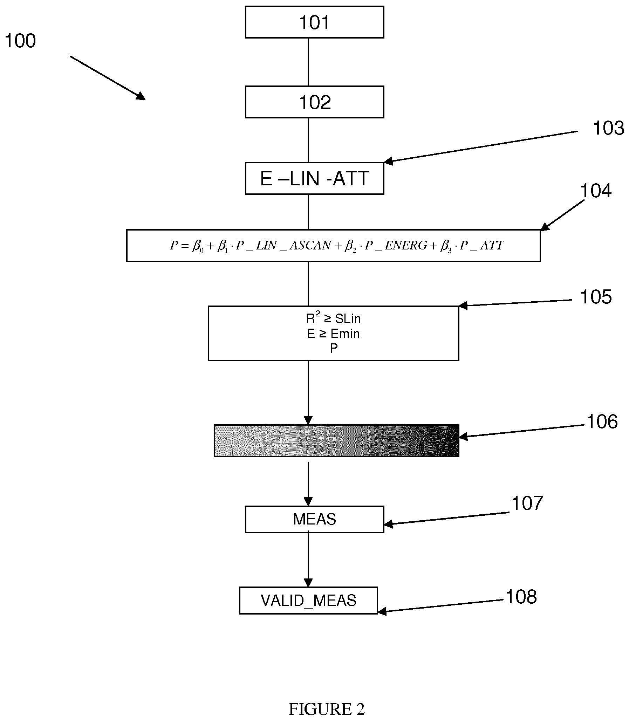

Les étapes du procédé 100 pour déterminer en temps-réel une probabilité de présence d'un tissu biologique cible en regard d'un transducteur ultrasonore sont notamment décrites à l'appui des figures 1 à 3.

Dans un exemple non limitatif de mise en oeuvre d'un procédé 100 conforme à l'invention, le tissu biologique cible est le parenchyme hépatique. Ainsi, préalablement à la mise en oeuvre du procédé 100, on positionne approximativement le transducteur ultrasonore 2 en regard du parenchyme hépatique 6.

Dans une première étape, dès lors que le transducteur ultrasonore 2 est approximativement positionné, on déclenche une émission 101 via le transducteur ultrasonore 2, d'un signal ultrasonore dans le tissu biologique situé en regard du transducteur ultrasonore 2. La fréquence d'émission de ce transducteur ultrasonore 2 peut par exemple être de 2,5 MHz.

Dans une deuxième étape, une réception 102, par le transducteur ultrasonore 2, du signal ultrasonore émis rétrodiffusé par le tissu biologique situé en regard du transducteur ultrasonore 2 est réalisée.

Dans une troisième étape, un calcul 103 d'au moins deux paramètres instantanés du signal ultrasonore rétrodiffusé reçu lors de l'étape de réception 102 est réalisé.

Dans un exemple non limitatif, on calcule 103 deux des trois paramètres suivants :

  ∘ l'énergie E du signal ultrasonore rétrodiffusé,
  ∘ l'atténuation ATT du signal ultrasonore rétrodiffusé,
  ∘ la linéarité LIN de l'enveloppe du signal ultrasonore rétrodiffusé.
  ∘ le coefficient d'intercorrélation des signaux ultrasonores rétrodiffusés reçus à deux moments successifs,
  ∘ l'amplitude ou les variations maximum d'amplitude de l'enveloppe du signal ultrasonore rétrodiffusé,
  ∘ la corrélation du au moins un signal ultrasonore rétrodiffusé,
  ∘ la variance spectrale du au moins un signal ultrasonore rétrodiffusé,
  ∘ le coefficient de rétrodiffusion du au moins un signal ultrasonore rétrodiffusé.

Il est entendu que les paramètres calculés précités sont non limitatifs. Ainsi, d'autres paramètres peuvent être calculés.

Dans un exemple avantageux non limitatif, on calcul 103 les trois paramètres suivants :

  ∘ l'énergie E du signal ultrasonore rétrodiffusé,
  ∘ l'atténuation ATT du signal ultrasonore rétrodiffusé, et
  ∘ la linéarité LIN de l'enveloppe du signal ultrasonore rétrodiffusé.

Il convient de noter que le choix des trois paramètres n'est pas limité à ceux précités, à savoir l'énergie E, l'atténuation ATT et la linéarité LIN de l'enveloppe du signal ultrasonore rétrodiffusé.

Le paramètre de l'énergie E du signal ultrasonore.

Dans un exemple non limitatif, le paramètre de l'énergie E du signal ultrasonore rétrodiffusé correspondant à l'amplitude du signal ultrasonore rétrodiffusé au carré (également dénommée par l'acronyme IBS pour Integrated BackScatter en anglais) peut être calculé au moyen de la formule suivante :

$$IBS = \sum_{Z\min}^{Z\max} Signal(z)^2$$

Avec :

- z = vt/2, où v est la vitesse des ultrasons dans le milieu et t le temps mis par le signal ultrasonore pour parcourir la distance z, signal = amplitude du signal ultrasonore rétrodiffusé.

Le paramètre de l'atténuation ATT du signal ultrasonore rétrodiffusé.

Dans un exemple non limitatif, le paramètre de l'atténuation ATT du signal ultrasonore rétrodiffusé est calculé au moyen de la formule suivante : $ATT(f,z) = e^{\alpha(f)z}$

Dans laquelle, le coefficient $\alpha(f)$ est calculé au moyen de la formule suivante :

$$\alpha(f) = \frac{1}{e} \ln \frac{P_1(f)}{P_2(f)} \qquad Np.m^{-1}$$

Avec :

- e = épaisseur du tissu biologique traversé par un signal ultrasonore émis,
- $P_1$ = énergie du signal ultrasonore émis, et
- $P_2$ = énergie du signal ultrasonore après avoir traversé l'épaisseur e de mesure du tissu biologique.

En outre dans le document (Pauly H, Schwan P., Mechanism of absorption of ultrasound in liver tissue. J.Acoust. Soc. Am., 1971. 50(2B), pp. 692-699), il a été démontré que l'atténuation ATT du signal ultrasonore rétrodiffusé décroit quasi linéairement avec la fréquence. En d'autres termes, dans le domaine des ultrasons, une mesure d'atténuation définie le calcul de la pente d'atténuation exprimée en dB.cm$^{-1}$.MHz$^{-1}$. Ainsi, dans un autre exemple non limitatif, l'atténuation ATT du signal ultrasonore rétrodiffusé est calculée via la formule suivante :

$$ATT = \alpha(f) = \alpha_0 + \beta(f - f_c) \qquad dB.cm^{-1}$$

Avec :

- $\beta$ (dB.cm$^{-1}$.MHz$^{-1}$) = la pente d'atténuation en fonction de la fréquence d'émission du transducteur ultrasonore,
- f = la fréquence d'émission du transducteur ultrasonore,
- fc = la fréquence centrale du transducteur ultrasonore, et
- $\alpha_0$ (dB.cm$^{-1}$) = l'atténuation à la fréquence centrale fc.

Dans un exemple non limitatif, les coefficients $\alpha_0$ et $\beta$ sont prédéterminés de façon empirique.

La linéarité LIN de l'enveloppe du signal ultrasonore rétrodiffusé.

La linéarité LIN de l'enveloppe du signal ultrasonore rétrodiffusé est performante pour détecter une interface dans un signal linéairement atténué.

Dans un exemple non limitatif appliqué au foie, dans un milieu homogène, l'A-SCAN qui représente le logarithme de l'enveloppe du signal ultrasonore rétrodiffusé, possède une composante linéaire. Cette composante est la valeur de l'atténuation dans le domaine temporel. La linéarité LIN de l'enveloppe du signal ultrasonore rétrodiffusé peut par exemple être utilisée pour détecter une interface formée par des vaisseaux sanguins ou par la paroi des intestins. La détection de la paroi des intestins se traduit par un pic plus ou moins large sur l'A-SCAN. Dans les deux cas, la linéarité de l'A-SCAN n'est plus respectée.

Le problème majeur est de trouver un paramètre représentant cette linéarité : le coefficient de détermination $R^2$ obtenu par régression linéaire est adapté pour la caractériser.

Dans un exemple non limitatif, on coupe le signal ultrasonore rétrodiffusé en un nombre N de fenêtres, on moyenne ces fenêtres et on calcule, sur ces moyennes, le coefficient de détermination $R^2$ ou le RMSE (dénommé Root Mean Squared Error en anglais).

Pour ce faire, on divise le signal *s* en N fenêtres de taille M avec $y_i$ correspondant à la moyenne des fenêtres :

$$y_i = \frac{1}{M} \sum_{j=1}^{M} s(i,j) \qquad i = [1, N]$$

On applique ensuite une régression linéaire au sens des moindres carrés : $y_i = ax_i + b$ avec x la profondeur en millimètres. La régression linéaire consiste à déterminer une estimation des valeurs a (coefficient directeur) et b (ordonnée à l'origine) et à quantifier la validité de cette relation grâce au coefficient de détermination $R^2$. Le coefficient de détermination $R^2$ est compris entre 0 et 1. Plus il est proche de 1 et plus la qualité globale de la régression linéaire est bonne. Il rend compte de la linéarité des points yi. Si les points $y_i$ sont parfaitement alignés, le modèle linéaire aura un coefficient

de détermination R$^2$ égal à 1. Au contraire, si les points sont très dispersés, l'écart des points à la droite du modèle linéaire sera important. Le coefficient de détermination tendra vers 0.

Le coefficient de détermination R$^2$ est calculé via la formule suivante :

$$R^2 = 1 - \frac{SCR}{SCT}$$

Avec :

- $SCR = \sum_i (y_i - \hat{y}_i)^2$ = la somme des carrés résiduels

- $SCT = \sum_i (y_i - \overline{y}_i)^2$ = la somme des carrés totaux

Dans un exemple non limitatif, le coefficient RMSE est calculé via la formule suivante : $RMSE = \frac{1}{N} SCR$

Lors d'une quatrième étape, on calcule 104 une valeur prédictive P de présence d'une signature acoustique d'un tissu biologique cible, la valeur prédictive P étant calculée via une loi statistique utilisant les au moins deux paramètres instantanés calculés, à savoir :

- l'énergie E du signal ultrasonore rétrodiffusé,
- l'atténuation ATT du signal ultrasonore rétrodiffusé,
- la linéarité LIN de l'enveloppe du signal ultrasonore rétrodiffusé.

Dans un exemple non limitatif, la loi statistique est une régression logistique. Selon une possibilité, la valeur prédictive P est calculée via le modèle de régression logistique suivant :

$$P = \beta_0 + \beta_1 \cdot Param\grave{e}tre\_calcul\acute{e}\_1 + \ldots\ldots + \beta_n \cdot Param\grave{e}tre\_calcul\acute{e}\_n$$

Dans l'exemple décrit, la valeur prédictive P est calculée via le modèle de régression logistique suivant :

$$P = \beta_0 + \beta_1 \cdot Param\grave{e}tre\_calcul\acute{e}\_1 + \beta_2 \cdot Param\grave{e}tre\_calcul\acute{e}\_2 + \beta_3 \cdot Param\grave{e}tre\_calcul\acute{e}\_3$$

Avec :

- Paramètre_calculé_1 = énergie E du signal ultrasonore rétrodiffusé,
- Paramètre_calculé_2 = atténuation ATT du signal ultrasonore rétrodiffusé, et
- Paramètre_calculé_3 = linéarité LIN de l'enveloppe du signal ultrasonore rétrodiffusé.

Dans une mise en oeuvre différente, le calcul 104 de la valeur prédictive P est réalisé via les au moins deux paramètres instantanés calculés et des paramètres instantanés calculés antérieurement mémorisés. Lors de cette mise en oeuvre, des paramètres instantanés calculés sont mémorisés sur une période de temps donnée.

Par exemple:

  ◦ au moment T-2, les paramètres instantanés de l'énergie E et de la linéarité LIN sont calculés et mémorisés ;
  ◦ au moment T-1, les paramètres instantanés de l'énergie E et de la linéarité LIN sont calculés et mémorisés ;
  ◦ au moment T, les paramètres instantanés de l'énergie E et de la linéarité LIN sont calculés.

Selon cette mise en oeuvre, la valeur prédictive P est ensuite calculée via les paramètres instantanés calculés et mémorisés au moment T-2 et au moment T-1 et les paramètres instantanées calculés au moment T. Cette particularité permet de prendre en considération les mouvements des tissus biologiques dus par exemple aux mouvements respiratoires.

Dans une mise en oeuvre différente, le calcul 104 de la valeur prédictive fait appel à un paramètre instantané calculé à partir du signal ultrasonore obtenu à différents moments. C'est le cas par exemple lorsque le coefficient d'intercorrélation entre les signaux ultrasonores aux moments T et T-1 est retenu dans les paramètres instantanés utilisés pour le calcul de la valeur prédictive.

Dans une cinquième étape, on estime 105 une probabilité de présence du tissu biologique cible en regard du transducteur ultrasonore 2, l'estimation 105 étant fonction de ladite valeur prédictive P calculée et/ou d'au moins une condition de robustesse basée sur au moins un des deux paramètres instantanés calculés E, LIN, ATT.

Plus particulièrement, l'estimation 105 fonction de la valeur prédictive P calculée est réalisée en comparant la valeur prédictive P calculée à une unique ou plusieurs valeurs seuil de prédiction déterminée de sorte que : lorsque la valeur prédictive P calculée est comparée à une unique valeur seuil de prédiction maximum $S_{Max}$ déterminée, alors

- si la valeur prédictive P calculée est strictement inférieure à la valeur seuil de prédiction maximum $S_{Max}$ déterminée alors le transducteur ultrasonore 2 est en regard du tissu biologique cible,
- si la valeur prédictive P calculée est supérieure ou égale à la valeur seuil de prédiction maximum $S_{Max}$ déterminée, alors le transducteur ultrasonore 2 est en regard d'un tissu biologique différent du tissu biologique cible ;
  lorsque la valeur prédictive P calculée est comparée à une valeur seuil de prédiction maximum $S_{Max}$ déterminée et une valeur seuil de prédiction minimum $S_{Min}$ déterminée, alors
- si la valeur prédictive P calculée est inférieure ou égale à la valeur seuil de prédiction minimum $S_{Min}$ déterminée alors le transducteur ultrasonore 2 est en regard du tissu biologique cible,
- si la valeur prédictive P calculée est supérieure ou égale à la valeur seuil de prédiction maximum $S_{Max}$ déterminée, alors le transducteur ultrasonore 2 est en regard d'un tissu biologique différent du tissu biologique cible,
- si la valeur prédictive P calculée est strictement supérieure à la valeur seuil de prédiction minimum $S_{Min}$ déterminée et strictement inférieure à la valeur seuil de prédiction maximum $S_{Max}$ déterminée alors le transducteur ultrasonore 2 est supposé être en regard du tissu biologique cible. Cette incertitude peut correspondre à une signature acoustique du tissu biologique cible modifiée par une présence d'hétérogénéités engendrée par des métastases ou encore par la présence de vaisseaux sanguins, etc.

Il convient de noter que pour un même tissu biologique comme par exemple le parenchyme hépatique, en fonction des fréquences ultrasonores émises par le transducteur ultrasonore 2, les valeurs seuils de prédiction minimum $S_{Min}$ et maximum $S_{Max}$ utilisées diffèrent.

Lors de la cinquième étape, on peut également estimer 105, fonction d'au moins une condition de robustesse basée sur au moins un des deux paramètres instantanés calculés E, LIN, ATT, une probabilité de présence du tissu biologique cible en regard du transducteur ultrasonore 2.

Plus particulièrement, la au moins une condition de robustesse peut être :

- si la linéarité LIN de l'enveloppe du signal ultrasonore rétrodiffusé calculée est supérieure ou égale à une valeur seuil de linéarité $S_{Lin}$ déterminée, alors le transducteur ultrasonore 2 est en regard du tissu biologique cible, et/ou
- si l'énergie E du signal ultrasonore rétrodiffusé est supérieure ou égale à une valeur seuil d'énergie $E_{Min}$ déterminée, alors le transducteur ultrasonore 2 est en regard du tissu biologique cible.

Dans un exemple non limitatif et comme indiqué précédemment la valeur représentative de la linéarité LIN de l'enveloppe du signal ultrasonore rétrodiffusé calculée peut être le coefficient de détermination $R^2$.

En revanche, quelque soit la valeur prédictive P calculée :

- si la linéarité LIN de l'enveloppe du signal ultrasonore rétrodiffusé calculée est strictement inférieure à la valeur seuil de linéarité $S_{Lin}$ déterminée, alors le transducteur ultrasonore 2 est en regard d'un tissu biologique différent du tissu biologique cible,
- si l'énergie E du signal ultrasonore rétrodiffusé est strictement inférieure à la valeur seuil d'énergie $E_{min}$ déterminée, alors le transducteur ultrasonore 2 est en regard d'un tissu biologique différent du tissu biologique cible.

Ainsi, il convient de noter que si au moins une des conditions de robustesse indique que le transducteur ultrasonore 2 n'est pas situé en regard du tissu biologique cible alors on ne tient pas compte de la valeur prédictive P calculée. En d'autres termes, si au moins une des conditions de robustesse indique que le transducteur ultrasonore 2 n'est pas situé en regard du tissu biologique cible alors l'estimation fonction de la valeur prédictive P calculée n'est pas prise en considération lors de l'estimation 105.

A titre d'exemple, l'estimation 105 fonction d'au moins une condition de robustesse basée sur la linéarité LIN du signal ultrasonore rétrodiffusé permet de vérifier qu'il n'y a pas d'hétérogénéité importante comme par exemple la présence de vaisseaux et de vérifier également que le parenchyme hépatique est situé en regard du transducteur ultrasonore.

A titre d'exemple, l'estimation 105 fonction d'au moins une condition de robustesse basée sur l'énergie E du signal ultrasonore rétrodiffusé permet de vérifier que le transducteur ultrasonore 2 est couplé correctement au milieu étudié ou qu'il n'est pas situé en regard d'une partie dure (os). Lorsque le transducteur ultrasonore 2 n'est pas couplé correctement avec le milieu (transducteur ultrasonore en contact avec un os ou pas en contact), l'énergie E du signal ultrasonore rétrodiffusé est très faible.

Il convient de noter que la valeur seuil d'énergie minimum $E_{Min}$ déterminée et la valeur seuil de linéarité minimum $S_{Lin}$ déterminée sont déterminées empiriquement par l'analyse de données.

Il convient de noter que pour un même tissu biologique comme par exemple le parenchyme hépatique, en fonction des fréquences ultrasonores émises par le transducteur ultrasonore 2, la valeur seuil d'énergie minimum $E_{Min}$ déterminée et la valeur seuil de linéarité minimum $S_{Lin}$ déterminée utilisées diffèrent.

Ainsi, la valeur seuil d'énergie minimum $E_{Min}$ déterminée et la valeur seuil de linéarité minimum $S_{Lin}$ déterminée utilisées dans les conditions de robustesse pour un transducteur ultrasonore 2 émettant une fréquence ultrasonore de 2,5 MHz seront différentes d'une valeur seuil d'énergie minimum $E_{Min}$ et d'une valeur seuil de linéarité minimum $S_{Lin}$ utilisées dans les conditions de robustesse pour un transducteur ultrasonore 2 émettant une fréquence ultrasonore de 3,5 MHz.

Dans une mise en oeuvre de l'invention, le procédé comporte une étape supplémentaire d'indication 106 en temps-réel d'une probabilité de présence du tissu biologique cible en regard du transducteur ultrasonore 2.

L'indication 106 peut être visuelle et/ou sonore et/ou tactile. L'indication visuelle peut être une indication paramétrique (c'est-à-dire sous forme d'une valeur numérique) ou colorimétrique. L'indication sonore peut être effectuée au moyen d'un bip retentissant lorsque le transducteur ultrasonore 2 n'est pas situé en regard du tissu biologique cible, ou inversement.

Dans un exemple non limitatif, lorsque l'indication 106 est visuelle, l'indicateur permettant d'indiquer la probabilité de présence du tissu biologique cible en regard du transducteur ultrasonore 2 peut être représenté sous la forme d'un feu tricolore rouge - orange - vert :

- la couleur rouge indiquant que le transducteur ultrasonore 2 n'est pas situé en regard du tissu biologique cible,
- la couleur orange indiquant que le transducteur ultrasonore 2 est probablement situé en regard du tissu biologique cible, dans ce cas une mesure 107 quantitative et/ou qualitative du tissu biologique cible peut être déclenchée automatiquement ou manuellement par un opérateur,
- la couleur verte indiquant que le transducteur ultrasonore 2 est situé en regard du tissu biologique cible, dans ce cas une mesure 107 quantitative et/ou qualitative du tissu biologique cible peut être déclenchée automatiquement ou manuellement par un opérateur.

Grâce à cette indication 106, la couleur varie en temps réel en fonction du déplacement du transducteur ultrasonore 2. L'indication 106 permet ainsi de déterminer si le transducteur ultrasonore 2 est situé en regard du tissu biologique.

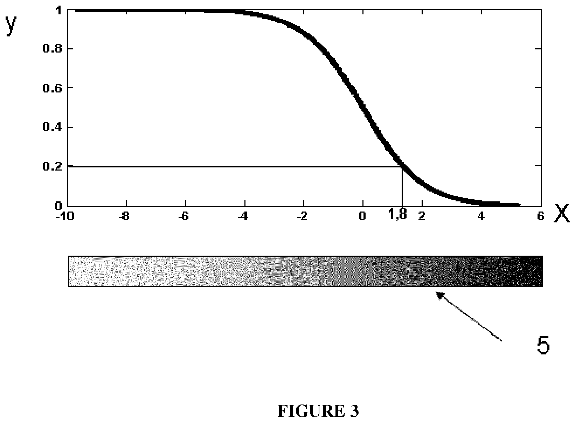

Dans une mise en oeuvre différente de l'invention illustrée sur la figure 3, l'indication 106 visuelle est réalisée par un affichage d'un gradient de couleur, en l'occurrence la couleur grise. Préférentiellement le gradient de couleur est de couleur verte. Le gradient de couleur vert permet d'obtenir une multitude de couleurs vertes différentes variant en fonction du tissu biologique situé en regard du transducteur ultrasonore 2. A titre d'exemple, si la couleur d'affichage est le vert clair alors le transducteur ultrasonore 2 est situé en regard du tissu biologique cible. Au contraire, si la couleur d'affichage est le vert foncé voire le noir alors le transducteur ultrasonore 2 est situé en regard d'un tissu biologique différent du tissu biologique cible.

Un tel gradient de couleur peut par exemple suivre une courbe en S, soumise à une loi de transparence de type :

$$y = 1 - \frac{1}{1 + e^{-x}} \qquad avec \qquad x = \text{Pestimée\_105} + S_{Min}$$

Dans cette équation :

- lorsque la au moins une condition de robustesse n'est pas prise en considération lors de l'étape d'estimation 105 d'une probabilité de présence du tissu biologique cible en regard du transducteur ultrasonore 2 alors $P_{estimée\_105}$ = valeur prédictive P calculée lors de l'étape 104,
- lorsque la au moins une condition de robustesse est prise en considération lors de l'étape d'estimation 105 d'une probabilité de présence du tissu biologique cible en regard du transducteur ultrasonore 2 alors $P_{estimée\_105}$ tient compte de la valeur prédictive P calculée lors de l'étape 104 et d'un au moins des deux paramètres instantanés calculés, à savoir dans l'exemple décrit du coefficient de détermination $R^2$ (ou linéarité LIN du signal ultrasonore rétrodiffusé) et de l'énergie E du signal ultrasonore rétrodiffusé. Si au moins un des deux paramètres indique que le transducteur ultrasonore n'est pas situé en regard du tissu biologique cible alors $P_{estimée\_105}$ est considéré comme étant égale à la valeur seuil de prédiction maximum $S_{max}$. Cette hypothèse indique que le transducteur ultrasonore 2 n'est pas situé en regard du tissu biologique cible.

Sur la figure 3, l'axe des abscisses x représente l'estimation d'une probabilité $P_{estimée\_105}$ de présence du tissu biologique cible en regard dudit transducteur ultrasonore (x de la loi de transparence) et l'axe des ordonnées y représente « y » de la loi de transparence.

Cette loi de transparence possède pour objectif d'adapter la transition entre une couleur opaque (par exemple gris opaque sur la figure 3 ou selon un autre exemple non représenté vert opaque) et la même couleur mais transparente (par exemple, gris transparent ou vert transparent). La pente de cette courbe est la zone de transition entre la couleur opaque et la couleur transparente. Cette zone de transition peut être adaptée suivant le compromis entre une présence du tissu biologique cible dite optimale d'une présence du tissu biologique dite intermédiaire suivant ce que l'on recherche. Par optimale, on entend une forte probabilité d'être en regard du tissu biologique cible. Par intermédiaire, on entend une probabilité moyenne plus faible d'être en regard du tissu biologique cible.

En résumé, dans l'exemple décrit, plus la transparence est importante et plus la couleur est claire. Inversement, plus la transparence est faible et plus la couleur est sombre. Il est entendu que la couleur verte ou la couleur grise ne sont pas limitatives.

A titre d'exemple, pour une valeur d'estimation $P_{estimée\_105}$ d'une probabilité de présence du tissu biologique cible en regard dudit transducteur ultrasonore égale à 1,8, la transparence y correspondante est égale à 0, 2. Comme la valeur y de la transparence est faible, la couleur d'indication 106 obtenue représentée sur l'indicateur 5 est sombre.

Par analogie avec l'exemple d'indication 106 précédent, le vert opaque correspond au feu vert, le vert de transition au feu orange et le vert transparent au feu rouge.

Une telle indication est compréhensible par la majorité de la population sans nécessité des connaissances accrues dans le domaine de la médecine. En effet, lorsque l'opérateur perçoit une couleur verte, il sait que le transducteur ultrasonore 2 est situé en regard du tissu biologique cible.

Comme indiqué précédemment, le procédé 100 comporte également une étape de mesure (MEAS) 107 quantitative et/ou qualitative du tissu biologique cible, cette mesure 107 pouvant être déclenchée automatiquement ou manuellement par un opérateur lorsque l'indication 106 indique que le transducteur ultrasonore 2 est situé en regard du tissu biologique cible. Dans le cas du foie, la mesure 107 est réalisée, par exemple, sur une profondeur PF comprise entre 25 mm et 80 mm, la profondeur PF étant mesurée à partir de la surface de contact entre ledit transducteur ultrasonore 2 et le tissu biologique 6 en contact. Par exemple, la profondeur PF peut être comprise entre :

- 35 et 75 mm pour une fréquence d'émission de l'ordre de 2.5 MHz,
- 25 et 65 mm pour une fréquence d'émission de l'ordre de 3.5 MHz, ou
- 15 et 55 mm pour une fréquence d'émission de l'ordre de 6 MHz.

Au cours de cette étape, on peut mesurer l'élasticité des tissus biologiques, la viscosité des tissus biologiques, le CAP (controlled Atténuation Parameter en anglais) ou tout autre paramètre quantitatif issu des signaux ultrasonores caractérisant les tissus biologiques mous.

Le procédé 100 comporte également une étape de validation (VALID_MEAS) 108 de la mesure 107 quantitative et/ou qualitative. Cette étape de validation 108 permet de s'assurer que la mesure obtenue est une mesure représentative du tissu biologique cible.

De façon générale, le procédé 100 de l'invention permet notamment de :

- déterminer en temps-réel si le transducteur ultrasonore est positionné en regard d'un tissu biologique cible,
- quantifier la qualité du signal ultrasonore rétrodiffusé par le tissu biologique,
- réaliser une mesure en 2D (deux dimensions) ou 3D (3 dimensions) et plus avantageusement, uniquement en 1D (une dimension, c'est-à-dire sur une ligne ultrasonore) ; en effet une mesure en 2D ou 3D n'est pas nécessaire, de ce fait le traitement des données du signal ultrasonore rétrodiffusé est plus rapide,

- considérer la sensibilité et la fréquence des transducteurs ultrasonores, c'est-à-dire que pour un transducteur ultrasonore spécifique la sensibilité et la fréquence du transducteur ultrasonore spécifique fournies par le fabricant sont prises en considération de façon à adapter le signal émis par ce transducteur ultrasonore spécifique à la loi statistique ;
- détecter une erreur due à l'opérateur par exemple un manque de couplant ultrasonore ou gel ultrasonore (signal ultrasonore faible) ;
- détecter une défaillance technique par exemple un problème de connectique générant une perte de signal ultrasonore ;
- d'être utilisé comme critère de validation d'une mesure ou d'indice de confiance sur la mesure réalisée.

Le procédé 100 de l'invention a plus particulièrement été décrit dans le cas d'une application au parenchyme hépatique (ou foie). Toutefois, il est entendu qu'un tel procédé peut être appliqué à tout type de tissu biologique. Pour ce faire, il faut recalculer des coefficients et valeurs seuils pertinents vis-à-vis de ce nouveau tissu biologique cible.

L'invention porte également sur dispositif 1 (Cf. figure 1) pour déterminer en temps-réel une probabilité de présence d'un tissu biologique cible 6 en regard d'un transducteur ultrasonore 2.

Comme indiqué précédemment, le dispositif 1 comporte un transducteur ultrasonore 2 relié à une unité de traitement des informations 3 par une liaison filaire 4. Le dispositif 1 comporte en outre un indicateur 5 en temps réel d'une probabilité de présence d'un tissu biologique cible en regard du transducteur ultrasonore 2.

Le dispositif 1 est apte à :

- émettre 101, via le transducteur ultrasonore 2, un signal ultrasonore dans un tissu biologique ;
- recevoir 102, par le transducteur ultrasonore 2, le signal ultrasonore émis rétrodiffusé par le tissu biologique ;
- calculer 103 au moins deux paramètres instantanés E, LIN, ATT du signal ultrasonore rétrodiffusé ;
- calculer 104 une valeur prédictive P de présence d'une signature acoustique d'un tissu biologique cible, la valeur prédictive P étant calculée via une loi statistique utilisant les au moins deux paramètres instantanés E, LIN, ATT calculés ;
- estimer 105 une probabilité de présence du tissu biologique cible en regard du transducteur ultrasonore 2, l'estimation 105 étant fonction de la valeur prédictive P et/ou d'au moins une condition de robustesse basée sur au moins un des deux paramètres instantanés E, LIN, ATT calculés,
- indiquer 106, via un indicateur 5, en temps réel une probabilité de présence du tissu biologique cible en regard du transducteur ultrasonore 2.

L'indicateur 5 permet d'indiquer en temps-réel à l'opérateur utilisant le dispositif 1 si le transducteur ultrasonore 2 est positionné en regard du tissu biologique cible. Ainsi, l'utilisation d'un tel dispositif 1 ne requiert aucune compétence dans le domaine médical ni même de l'instrumentation médicale. Comme indiqué précédemment l'indicateur 5 peut être sous la forme d'un feu tricolore, d'un gradient de couleur, d'une couleur soumise à une loi de transparence ou encore d'une gamme de couleur (i.e. palette de couleur).

En outre, le dispositif 1 pour déterminer en temps-réel une probabilité de présence d'un tissu biologique cible 6 en regard d'un transducteur ultrasonore 2 peut être un élastographe, un échographe ou plus généralement tout dispositif utilisant des ultrasons. Ainsi, un tel dispositif 1 permet non seulement de positionner le transducteur ultrasonore 2 en regard d'un tissu biologique cible mais également d'effectuer une mesure quantitative et/ou qualitative du tissu biologique cible.

## Revendications

1. Procédé (100) pour déterminer en temps-réel une probabilité de présence d'un tissu biologique cible en regard d'un transducteur ultrasonore (2), ledit procédé (100) comportant les étapes suivantes :

   - émission (101), via un transducteur ultrasonore (2), d'au moins un signal ultrasonore dans un tissu biologique ;
   - réception (102), par ledit transducteur ultrasonore (2), dudit au moins un signal ultrasonore émis rétrodiffusé par ledit tissu biologique ;
   - calcul (103) d'au moins deux paramètres (E, LIN, ATT) instantanés dudit au moins un signal ultrasonore rétrodiffusé ;
   - calcul (104) d'une valeur prédictive (P) de présence d'une signature acoustique d'un tissu biologique cible, ladite valeur prédictive (P) étant calculée via une loi statistique utilisant lesdits au moins deux paramètres instantanés calculés (E, LIN, ATT) ;
   - estimation (105) d'une probabilité de présence dudit tissu biologique cible en regard dudit transducteur ultrasonore (2), ladite estimation (105) étant fonction de ladite valeur prédictive (P) calculée et d'au moins une condition de robustesse basée sur au moins un desdits deux paramètres instantanés calculés (E, LIN, ATT).

2. Procédé (100) selon la revendication 1 **caractérisé en ce que** ledit calcul (104) d'une valeur prédictive (P) est réalisé via lesdits au moins deux paramètres instantanés calculés (E, LIN, ATT) et des paramètres instantanés calculés (E, LIN, ATT) antérieurement mémorisés.

3. Procédé (100) selon l'une des revendications 1 ou 2 **caractérisé en ce que** ladite estimation (105) fonction de ladite valeur prédictive (P) calculée est réalisée en comparant ladite valeur prédictive (P) calculée à une unique ou plusieurs valeurs seuil de prédiction déterminée de sorte que :

lorsque la valeur prédictive (P) calculée est comparée à une unique valeur seuil de prédiction maximum ($S_{Max}$) déterminée,

- si la valeur prédictive (P) calculée est strictement inférieure à la valeur seuil de prédiction maximum ($S_{Max}$) déterminée alors le transducteur ultrasonore (2) est en regard du tissu biologique cible,
- si la valeur prédictive (P) calculée est supérieure ou égale à la valeur seuil de prédiction maximum ($S_{Max}$) déterminée, alors le transducteur ultrasonore (2) est en regard d'un tissu biologique différent du tissu biologique cible ;

lorsque la valeur prédictive (P) calculée est comparée à une valeur seuil de prédiction maximum ($S_{Max}$) déterminée et une valeur seuil de prédiction minimum ($S_{Min}$) déterminée,

- si la valeur prédictive (P) calculée est inférieure ou égale à la valeur seuil de prédiction minimum ($S_{Min}$) déterminée alors le transducteur ultrasonore (2) est en regard du tissu biologique cible,
- si la valeur prédictive (P) calculée est supérieure ou égale à la valeur seuil de prédiction maximum ($S_{Max}$) déterminée, alors le transducteur ultrasonore (2) est en regard d'un tissu biologique différent du tissu biologique cible,
- si la valeur prédictive (P) calculée est strictement supérieure à la valeur seuil de prédiction minimum ($S_{Min}$) déterminée et strictement inférieure à la valeur seuil de prédiction maximum ($S_{Max}$) déterminée alors le transducteur ultrasonore (2) est supposé être en regard du tissu biologique cible.

4. Procédé (100) selon l'une des revendications précédentes **caractérisé en ce que** lesdits au moins deux paramètres (E, LIN, ATT) instantanés calculés sont choisis parmi les paramètres suivants :

  ◦ l'énergie (E) dudit au moins un signal ultrasonore rétrodiffusé,
  ◦ l'atténuation (ATT) dudit au moins un signal ultrasonore rétrodiffusé,
  ◦ la linéarité (LIN) de l'enveloppe dudit au moins un signal ultrasonore rétrodiffusé.

5. Procédé (100) selon la revendication 4 **caractérisé en ce que** ladite condition de robustesse est :

  - si la linéarité (LIN) de l'enveloppe dudit au moins un signal ultrasonore rétrodiffusé calculée est supérieure ou égale à une valeur seuil de linéarité ($S_{Lin}$) déterminée, alors ledit transducteur ultrasonore (2) est en regard dudit tissu biologique cible, et/ou
  - si l'énergie (E) dudit au moins un signal ultrasonore rétrodiffusé est supérieure ou égale à une valeur seuil d'énergie ($E_{Min}$) déterminée, alors ledit transducteur ultrasonore (2) est en regard dudit tissu biologique cible.

6. Procédé (100) selon la revendication 4 **caractérisé en ce que** quelque soit ladite valeur prédictive (P) calculée, ladite condition de robustesse est :

  - si ladite linéarité (LIN) de l'enveloppe dudit au moins un signal ultrasonore rétrodiffusé calculée est strictement inférieure à une valeur seuil de linéarité ($S_{Lin}$) déterminée, alors ledit transducteur ultrasonore (2) est en regard d'un tissu biologique différent dudit tissu biologique cible,
  - si ladite énergie (E) dudit au moins un signal ultrasonore rétrodiffusé est strictement inférieure à une valeur seuil d'énergie ($E_{min}$) déterminée, alors ledit transducteur ultrasonore (2) est en regard d'un tissu biologique différent dudit tissu biologique cible.

7. Procédé (100) selon l'une des revendications précédentes **caractérisé en ce que** ladite loi statistique est une régression logistique de sorte que ladite valeur prédictive (P) soit calculée via ladite régression logistique suivante :

$$P = \beta_0 + \beta_1 \cdot Param\grave{e}tre\_calcul\acute{e}\_1 + ..... + \beta_n \cdot Param\grave{e}tre\_calcul\acute{e}\_n$$

8. Procédé (100) selon l'une des revendications précédentes **caractérisé en ce qu'**il comporte une étape supplémentaire d'indication (106) en temps réel d'une probabilité de présence du tissu biologique cible en regard dudit transducteur ultrasonore (2).

9. Procédé (100) selon la revendication 8 **caractérisé en ce que** ladite indication (106) est une indication :

○ visuelle, et/ou

○ sonore et/ou

○ tactile.

**10.** Procédé (100) selon la revendication 9 **caractérisé en ce que** ladite indication visuelle est réalisée par un affichage d'un gradient de couleur.

**11.** Procédé (100) selon la revendication 10 **caractérisé en ce que** ledit gradient de couleur suit une courbe en S.

**12.** Procédé (100) selon l'une des revendications précédentes **caractérisé en ce que** lorsque ledit transducteur ultrasonore (2) est en regard dudit tissu biologique cible, une mesure (107) quantitative et/ou qualitative dudit tissu biologique cible est déclenchée de façon automatique ou manuelle.

**13.** Procédé (100) selon la revendication 12 **caractérisé en ce que** la mesure (107) est réalisée sur une profondeur (PF) déterminée dépendant de la fréquence dudit au moins un signal ultrasonore émis, ladite profondeur (PF) étant mesurée à partir de la surface de contact entre ledit transducteur ultrasonore (2) et ledit tissu biologique en contact.

**14.** Procédé (100) selon l'une des revendications 12 ou 13 **caractérisé en ce qu'**il comporte une étape supplémentaire de validation (108) de ladite mesure (107) quantitative et/ou qualitative.

**15.** Dispositif (1) pour déterminer en temps-réel une probabilité de présence d'un tissu biologique cible en regard d'un transducteur ultrasonore, ledit dispositif (1) comportant au moins un transducteur ultrasonore (2) apte à émettre et recevoir des signaux ultrasonores, ledit dispositif (1) étant apte à :

- émettre (101), via ledit transducteur ultrasonore (2), au moins un signal ultrasonore dans un tissu biologique ;
- recevoir (102), par ledit transducteur ultrasonore (2), ledit au moins un signal ultrasonore émis rétrodiffusé par ledit tissu biologique ;
- calculer (103) au moins deux paramètres instantanés (E, LIN, ATT) dudit au moins un signal ultrasonore rétrodiffusé ;
- calculer (104) une valeur prédictive (P) de présence d'une signature acoustique d'un tissu biologique cible, ladite valeur prédictive (P) étant calculée via une loi statistique utilisant lesdits au moins deux paramètres instantanés (E, LIN, ATT) calculés ;
- estimer (105) une probabilité de présence dudit tissu biologique cible en regard dudit transducteur ultrasonore (2), ladite estimation (105) étant fonction de ladite valeur prédictive (P) et d'au moins une condition de robustesse basée sur au moins un desdits deux paramètres instantanés (E, LIN, ATT) calculés,
- indiquer (106), via un indicateur (7), en temps réel une probabilité de présence dudit tissu biologique cible en regard dudit transducteur ultrasonore (2).

**Patentansprüche**

**1.** Verfahren (100) zur Echtzeitbestimmung einer Vorhandenswahrscheinlichkeit eines biologischen Zielgewebes gegenüber einem Ultraschallwandler (2), wobei das Verfahren (100) die folgenden Schritte umfasst:

- Senden (101), über einen Ultraschallwandler (2), mindestens eines Ultraschallsignals in ein biologisches Gewebe,
- Empfangen (102), durch den Ultraschallwandler (2), des mindestens einen gesendeten, von dem biologischen Gewebe zurückgestreuten Ultraschallsignals,
- Berechnen (103) von mindestens zwei Momentparametern (E, LIN, ATT) des mindestens einen zurückgestreuten Ultraschallsignals,
- Berechnen (104) eines prädiktiven Vorhandenswerts (P) einer akustischen Signatur eines biologischen Zielgewebes, wobei der prädiktive Wert (P) über ein statistisches Gesetz berechnet wird, das die mindestens zwei berechneten Momentparameter (E, LIN, ATT) nutzt,
- Schätzen (105) einer Vorhandenswahrscheinlichkeit des biologischen Zielgewebes gegenüber dem Ultraschallwandler (2), wobei die Schätzung (105) von dem berechneten prädiktiven Wert (P) und/oder von mindestens einer Robustheitsbedingung, basierend auf mindestens einem der zwei berechneten Momentparameter (E, LIN, ATT), abhängig ist.

2. Verfahren (100) nach Anspruch 1, **dadurch gekennzeichnet, dass** die Berechnung (104) eines prädiktiven Werts (P) über die mindestens zwei berechneten Momentparameter (E, LIN, ATT) und zuvor gespeicherte berechnete Momentparameter (E, LIN, ATT) durchgeführt wird.

3. Verfahren (100) nach einem der Ansprüche 1 oder 2, **dadurch gekennzeichnet, dass** die Schätzung (105) als Funktion des berechneten prädiktiven Werts (P) durch Vergleichen des berechneten prädiktiven Werts (P) mit einem einzigen oder mehreren bestimmten Prädiktionsgrenzwerten durchgeführt wird, so dass:

wenn der berechnete prädiktive Wert (P) mit einem einzigen bestimmten maximalen Prädiktionsgrenzwert ($S_{Max}$) verglichen wird,

- wenn der berechnete prädiktive Wert (P) kleiner als der bestimmte maximale Prädiktionsgrenzwert ($S_{Max}$) ist, dann ist der Ultraschallwandler (2) gegenüber dem biologischen Zielgewebe,
- wenn der berechnete prädiktive Wert (P) größer oder gleich dem bestimmten maximalen Prädiktionsgrenzwert ($S_{Max}$) ist, dann ist der Ultraschallwandler (2) gegenüber einem biologischen Gewebe, das sich von dem biologischen Zielgewebe unterscheidet,

wenn der berechnete prädiktive Wert (P) mit einem bestimmten maximalen Prädiktionsgrenzwert ($S_{Max}$) und einem bestimmten minimalen Prädiktionsgrenzwert ($S_{Min}$) verglichen wird,

- wenn der berechnete prädiktive Wert (P) kleiner oder gleich dem bestimmten berechneten Prädiktionsgrenzwert ($S_{Min}$) ist, dann ist der Ultraschallwandler (2) gegenüber dem biologischen Zielgewebe,
- wenn der berechnete prädiktive Wert (P) größer oder gleich dem bestimmten maximalen Prädiktionsgrenzwert ($S_{Max}$) ist, dann ist der Ultraschallwandler (2) gegenüber einem biologischen Gewebe, das sich von dem biologischen Zielgewebe unterscheidet,
- wenn der berechnete prädiktive Wert (P) größer als der berechnete minimale Prädiktionsgrenzwert ($S_{Min}$) und kleiner als der bestimmte maximale Prädiktionsgrenzwert ($S_{Max}$) ist, dann ist anzunehmen, dass der Ultraschallwandler (2) gegenüber dem biologischen Zielgewebe ist.

4. Verfahren (100) nach einem der vorangehenden Ansprüche, **dadurch gekennzeichnet, dass** die mindestens zwei berechneten Momentanparameter (E, LIN, ATT) aus den folgenden Parametern ausgewählt sind:

○ der Energie (E) des mindestens einen zurückgestreuten Ultraschallsignals,
○ der Dämpfung (ATT) des mindestens einen zurückgestreuten Ultraschallsignals,
○ der Linearität (LIN) der Hülle des mindestens einen zurückgestreuten Ultraschallsignals.

5. Verfahren (100) nach Anspruch 4, **dadurch gekennzeichnet, dass** die Robustheitsbedingung ist:

- wenn die berechnete Linearität (LIN) der Hülle des mindestens einen zurückgestreuten Ultraschallsignals größer oder gleich einem bestimmten Linearitätsgrenzwert ($S_{Lin}$) ist, dann ist der Ultraschallwandler (2) gegenüber dem biologischen Zielgewebe, und/oder
- wenn die Energie (E) des mindestens einen zurückgestreuten Ultraschallsignals größer oder gleich einem bestimmten Energiegrenzwert ($E_{Min}$) ist, dann ist der Ultraschallwandler (2) gegenüber dem biologischen Zielgewebe.

6. Verfahren (100) nach Anspruch 4, **dadurch gekennzeichnet, dass** unabhängig vom berechneten prädiktiven Wert (P) die Robustheitsbedingung ist:

- wenn die Linearität (LIN) der Hülle des mindestens einen berechneten zurückgestreuten Ultraschallsignals kleiner als ein bestimmter Linearitätsgrenzwert ($S_{Lin}$) ist, dann ist der Ultraschallwandler (2) gegenüber einem biologischen Gewebe, das sich von dem biologischen Zielgewebe unterscheidet,
- wenn die Energie (E) des mindestens einen zurückgestreuten Ultraschallsignals kleiner als ein bestimmter Energiegrenzwert ($E_{min}$) ist, dann ist der Ultraschallwandler (2) gegenüber einem biologischen Gewebe, das sich von dem biologischen Zielgewebe unterscheidet.

7. Verfahren (100) nach einem der vorangehenden Ansprüche, **dadurch gekennzeichnet, dass** das statistische Gesetz eine logistische Regression derart ist, dass der prädiktive Wert (P) über die folgende logistische Regression berechnet wird:

$$P = \beta_0 + \beta_1 \cdot berechneter\_Parameter\_1 + \ldots + \beta_n \cdot berechneter\_Parameter\_n$$

8. Verfahren (100) nach einem der vorangehenden Ansprüche, **dadurch gekennzeichnet, dass** es einen zusätzlichen Schritt der Angabe (106) in Echtzeit einer Vorhandenswahrscheinlichkeit des biologischen Zielgewebes gegenüber dem Ultraschallwandler (2) aufweist.

9. Verfahren (100) nach Anspruch 8, **dadurch gekennzeichnet, dass** die Angabe (106) eine Angabe ist:

   ○ visuell und/oder
   ○ akustisch und/oder
   ○ taktil.

10. Verfahren (100) nach Anspruch 9, **dadurch gekennzeichnet, dass** die visuelle Angabe durch eine Anzeige eines Farbgradienten durchgeführt wird.

11. Verfahren (100) nach Anspruch 10, **dadurch gekennzeichnet, dass** der Farbgradient eine S-Kurve beschreibt.

12. Verfahren (100) nach einem der vorangehenden Ansprüche, **dadurch gekennzeichnet, dass**, wenn der Ultraschallwandler (2) gegenüber dem biologischen Zielgewebe ist, eine quantitative und/oder qualitative Messung (107) des biologischen Zielgewebes automatisch oder manuell ausgelöst wird.

13. Verfahren (100) nach Anspruch 12, **dadurch gekennzeichnet, dass** die Messung (107) über eine bestimmte Tiefe (PF) durchgeführt wird, die von der Frequenz des mindestens einen gesendeten Ultraschallsignals abhängt, wobei die Tiefe (PF) ab der Kontaktfläche zwischen dem Ultraschallwandler (2) und dem kontaktierten biologischen Gewebe gemessen wird.

14. Verfahren (100) nach einem der Ansprüche 12 oder 13, **dadurch gekennzeichnet, dass** es einen zusätzlichen Schritt der Validierung (108) der quantitative und/oder qualitative Messung (107) aufweist.

15. Vorrichtung (1) zur Echtzeitbestimmung einer Vorhandenswahrscheinlichkeit eines biologischen Zielgewebes gegenüber einem Ultraschallwandler (2), wobei die Vorrichtung (1) mindestens einen Ultraschallwandler (2) aufweist, der imstande ist, Ultraschallsignale zu senden und zu empfangen, wobei die Vorrichtung (1) imstande ist zum:

   - Senden (101), über den Ultraschallwandler (2), mindestens eines Ultraschallsignals in ein biologisches Gewebe,
   - Empfangen (102), durch den Ultraschallwandler (2), des mindestens einen gesendeten, von dem biologischen Gewebe zurückgestreuten Ultraschallsignals,
   - Berechnen (103) von mindestens zwei Momentparametern (E, LIN, ATT) des mindestens einen zurückgestreuten Ultraschallsignals,
   - Berechnen (104) eines prädiktiven Vorhandenswerts (P) einer akustischen Signatur eines biologischen Zielgewebes, wobei der prädiktive Wert (P) über ein statistisches Gesetz berechnet wird, das die mindestens zwei berechneten Momentparameter (E, LIN, ATT) nutzt,
   - Schätzen (105) einer Vorhandenswahrscheinlichkeit des biologischen Zielgewebes gegenüber dem Ultraschallwandler (2), wobei die Schätzung (105) von dem berechneten prädiktiven Wert (P) und von mindestens einer Robustheitsbedingung, basierend auf mindestens einem der zwei berechneten Momentparameter (E, LIN, ATT), abhängig ist,
   - Angeben (106), über einen Indikator (7), in Echtzeit einer Vorhandenswahrscheinlichkeit des biologischen Zielgewebes gegenüber dem Ultraschallwandler (2).

**Claims**

1. A method (100) for determining in real time a probability of presence of a target biological tissue facing an ultrasonic transducer (2), said method (100) including the following steps of:

   - emitting (101), via an ultrasonic transducer (2), at least one ultrasound signal in a biological tissue;

- receiving (102), by said ultrasonic transducer (2), said at least one emitted ultrasound signal backscattered by said biological tissue;
- calculating (103) at least two instantaneous parameters (E, LIN, ATT) of said at least one backscattered ultrasound signal;
- calculating (104) a predictive value (P) of presence of an acoustic signature of a target biological tissue, said predictive value (P) being calculated via a statistical law using said at least two calculated instantaneous parameters (E, LIN, ATT);
- estimating (105) a probability of presence of said target biological tissue facing said ultrasonic transducer (2), said estimation (105) being a function of said calculated predictive value (P) and at least one robustness condition based on at least one of said two calculated instantaneous parameters (E, LIN, ATT).

2. The method (100) according to claim 1, **characterised in that** said calculation (104) of a predictive value (P) is made via said at least two calculated instantaneous parameters (E, LIN, ATT) and previously stored calculated instantaneous parameters (E, LIN, ATT).

3. The method (100) according to one of claims 1 and 2, **characterised in that** said estimation (105) as a function of said calculated predictive value (P) is made by comparing said calculated predictive value (P) with a single or several determined prediction threshold values such that:

when the calculated predictive value (P) is compared with a single determined maximum prediction threshold value ($S_{Max}$),

- if the calculated predictive value (P) is strictly lower than the determined maximum prediction threshold value ($S_{Max}$), then the ultrasonic transducer (2) is facing the target biological tissue,
- if the calculated predictive value (P) is higher than or equal to the determined maximum prediction threshold value ($S_{Max}$), then the ultrasonic transducer (2) is facing a biological tissue different from the target biological tissue;

when the calculated predictive value (P) is compared with a determined maximum prediction threshold value ($S_{Max}$) and a determined minimum prediction threshold value ($S_{Min}$),

- if the calculated predictive value (P) is lower than or equal to the determined minimum prediction threshold value ($S_{Min}$), then the ultrasonic transducer (2) is facing the target biological tissue,
- if the calculated predictive value (P) is higher than or equal to the determined maximum prediction threshold value ($S_{Max}$), then the ultrasonic transducer (2) is facing a biological tissue different from the target biological tissue,
- if the calculated predictive value (P) is strictly higher than the determined minimum prediction threshold value ($S_{Min}$) and strictly lower than the determined maximum prediction threshold value ($S_{Max}$), then the ultrasonic transducer (2) is supposed to be facing the target biological tissue.

4. The method (100) according to one of the preceding claims, **characterised in that** said at least two calculated instantaneous parameters (E, LIN, ATT) are chosen from the following parameters:

∘ the energy (E) of said at least one backscattered ultrasound signal,
∘ the attenuation (ATT) of said at least one backscattered ultrasound signal,
∘ the linearity (LIN) of the envelop of said at least one backscattered ultrasound signal.

5. The method (100) according to claim 4, **characterised in that** said robustness condition is:

- if the linearity (LIN) of the envelop of said at least one calculated backscattered ultrasound signal is higher than or equal to a determined linearity threshold value ($S_{Lin}$), then said ultrasonic transducer (2) is facing said target biological tissue, and/or
- if the energy (E) of said at least one backscattered ultrasound signal is higher than or equal to a determined energy threshold value ($E_{Min}$), then said ultrasonic transducer (2) is facing said target biological tissue.

6. The method (100) according to claim 4, **characterised in that** regardless of said calculated predictive value (P), said robustness condition is:

- if said linearity (LIN) of the envelop of said at least one calculated backscattered ultrasound signal is strictly lower than a determined linearity threshold value ($S_{Lin}$), then said ultrasonic transducer (2) is facing a biological tissue different from said target biological tissue,
- if said energy (E) of said at least one backscattered ultrasound signal is strictly lower than a determined energy threshold value ($E_{Min}$), then said ultrasonic transducer (2) is facing a biological tissue different from said target biological tissue.

7. The method (100) according to one of the preceding claims, **characterised in that** said statistical law is a logistic regression such that said predictive value (P) is calculated via said following logistic regression:

$$P = \beta_0 + \beta_1 \cdot Parameter\_calculated\_1 + \ldots + \beta_8 \cdot parameter\_calculated\_n$$

8. The method (100) according to one of the preceding claims, **characterised in that** it includes a further step of indicating (106) in real time a probability of presence of the target biological tissue facing said ultrasonic transducer (2).

9. The method (100) according to claim 8, **characterised in that** said indication (106) is:

   ◦ a visual indication, and/or
   ◦ a sound indication and/or
   ◦ a touch indication.

10. The method (100) according to claim 9, **characterised in that** said visual indication is performed by displaying a colour gradient.

11. The method (100) according to claim 10, **characterised in that** said colour gradient follows an S-curve.

12. The method (100) according to one of the preceding claims, **characterised in that** when said ultrasonic transducer (2) is facing said target biological tissue, a quantitative and/or qualitative measurement (107) of said target biological tissue is automatically or manually triggered.

13. The method (100) according to claim 12, **characterised in that** the measurement (107) is performed on a determined depth (PF) depending on the frequency of said at least one emitted ultrasound signal, said depth (PF) being measured from the contact surface between said ultrasonic transducer (2) and said biological tissue in contact therewith.

14. The method (100) according to one of claims 12 and 13, **characterised in that** it includes a further step of validating (108) said quantitative and/or qualitative measurement (107).

15. A device (1) for determining in real time a probability of presence of a target biological tissue facing an ultrasonic transducer, said device (1) including at least one ultrasonic transducer (2) able to emit and receive ultrasound signals, said device (1) being able to:

   - emit (101), via said ultrasonic transducer (2), at least one ultrasound signal in a biological tissue;
   - receive (102), by said ultrasonic transducer (2), said at least one emitted ultrasound signal backscattered by said biological tissue;
   - calculate (103) at least two instantaneous parameters (E, LIN, ATT) of said at least one backscattered ultrasound signal;
   - calculate (104) a predictive value (P) of presence of an acoustic signature of a target biological tissue, said predictive value (P) being calculated via a statistical law using said at least two calculated instantaneous parameters (E, LIN, ATT);
   - estimate (105) a probability of presence of said target biological tissue facing said ultrasonic transducer (2), said estimation (105) being a function of said predictive value (P) and of at least one robustness condition based on at least one of said two calculated instantaneous parameters (E, LIN, ATT).
   - indicate (106), via an indicator (7), in real time a probability of presence of said target biological tissue facing said ultrasonic transducer (2).

FIGURE 1

100

101

102

E –LIN -ATT  — 103

104

$$P = \beta_0 + \beta_1 \cdot P\_LIN\_ASCAN + \beta_2 \cdot P\_ENERG + \beta_3 \cdot P\_ATT$$

R$^2$ ≥ SLin
E ≥ Emin
P  — 105

106

MEAS  — 107

VALID_MEAS  — 108

FIGURE 2

FIGURE 3

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **SCHMITZ G et al.** Tissue Characterization of the Prostate Using Kohonen-Maps. *Ultrasonics Symposium,* 1994, 1487-1490

- **PAULY H ; SCHWAN P.** Mechanism of absorption of ultrasound in liver tissue. *J.Acoust. Soc. Am.,* 1971, vol. 50 (2B), 692-699